# EUROPÄISCHE PATENTSCHRIFT

(11) **EP 2 015 057 B1**
(45) Veröffentlichungstag und Bekanntmachung des Hinweises auf die Patenterteilung: **06.03.2019**
(21) Anmeldenummer: 07013639.5
(22) Anmeldetag: 12.07.2007
(51) Int. Cl.: G01N 25/28

(54) **Vorrichtung und Verfahren zur Detektion von Wasserstoff**
Device and method for detecting hydrogen
Dispositif et procédé destinés à la détection d'hydrogène

(43) Veröffentlichungstag der Anmeldung: 14.01.2009
(73) Patentinhaber: HOPPECKE Batterien GmbH & Co. KG, 59929 Brilon (DE)
(72) Erfinder: Kesper, Heinrich, 34508 Willingen (DE); Cattaneo, Eduardo, Dr., 59929 Brilon (DE); Riegel, Bernhard, Dr., 59929 Brilon (DE)
(74) Vertreter: Rausch Wanischeck-Bergmann Brinkmann

(56) Entgegenhaltungen:
- EP-A- 1 780 826
- DE-A1- 3 503 018
- DE-A1- 19 645 694
- DE-C1- 3 151 084
- GB-A- 761 055
- US-A- 5 483 228
- US-A1- 2003 056 570
- SHIN W ET AL: "Hydrogen-selective thermoelectric gas sensor" SENSORS AND ACTUATORS B, ELSEVIER SEQUOIA S.A., LAUSANNE, CH, Bd. 93, Nr. 1-3, 1. August 2003 (2003-08-01), Seiten 304-308, XP004437114 ISSN: 0925-4005

## Beschreibung

Die vorliegende Erfindung betrifft eine Vorrichtung sowie ein Verfahren zur Detektion von Wasserstoff in einem Gasvolumen.

Wasserstoff wird heutzutage kommerziell in breiten Anwendungsfeldern als Energieträger eingesetzt. So ist es beispielsweise bekannt, Brennstoffzellen mit Wasserstoff zu betreiben. Darüber hinaus ist es bekannt, Verbrennungsmotoren mit Wasserstoff zu betreiben. Wasserstoff birgt jedoch die Gefahr, dass bei eventuellen Leckagen von wasserstoffführenden Leitungen oder Systemen es zur Ausbildung von zündfähigen Luft/Wasserstoffgemischen kommen kann. Die Explosionsgrenze für Luft/Wasserstoffgemische reicht hierbei von 4 bis 75 Volumen-%. Solche zündfähigen bzw. explosiven Luft/Wasserstoffgemische werden auch als Knallgas bezeichnet.

Bei Wasserstoff handelt es sich um ein farb-, geruchs- und geschmacksloses Gas. Dies macht die Wahrnehmung von Wasserstoff durch die menschlichen Sinne äußerst schwierig bis unmöglich.

In Anbetracht eines Brennwertes von reinem Wasserstoff von 13 MJ/m³ stellen Leckagen in wasserstoffführenden Systemen ein immens großes Gefahrenpotential beim Betrieb von Wasserstoffanlagen dar.

Wasserstoff entsteht auch in Energiespeichern wie beispielsweise in Akkumulatoren, insbesondere während eines Ladevorganges. Auch hierbei kann es zur Ausbildung von explosiven Luft/Wasserstoffgemischen kommen.

Für den sicheren Betrieb von Wasserstoffanlagen oder Energiespeichern wie Akkumulatorbatterien ist es daher notwendig, die Ausbildung von zündfähigen Luft/Wasserstoffgemischen sicher bestimmen zu können, um entsprechende Gegenmaßnahmen wie beispielsweise das Belüften eines Raums oder das Unterbrechen der Wasserstoffzufuhr einleiten zu können.

Im Stand der Technik wird Wasserstoff bisher mittels elektrochemischer Sensoren mit Zwei- oder Dreielektrodenanordnung (Arbeitselektrode, Referenzelektrode und Gegenelektrode) (Bsp. US 7060169) detektiert. Die Sensoren enthalten eine wasserstoffdurchlässige Membran und werden für Messungen im Konzentrationsbereich unterhalb von 5 Vol.-% eingesetzt. Ein Nachteil von elektrochemischen Sensoren ist die Abnahme der Empfindlichkeit über die Zeit (Alterungseffekte). Dieser Effekt wird durch Degradierung des Elektrolyten sowie Nebenreaktionen an den Elektroden hervorgerufen. Durch Übersättigung des Elektrolyten bei sehr hohen Wasserstoffkonzentrationen, ist ebenfalls mit einer Lebensdauerverkürzung zu rechnen. In Anwesenheit von hohen Wasserdampfkonzentrationen können durch Kondensatbildung Korrosionserscheinungen an der Elektronik auf und/oder die Sperrung der Membran mit einer deutlichen Verlangsamung der Responszeit auftreten.

Ein weiteres Prinzip zur Wasserstoffdetektion nutzt eine Metalldünnschicht (z.B. Pd), wobei das Messprinzip auf einer Leitfähigkeitsänderungen (EP 768528) oder Lichttransmissionsänderung (EP 1521080) dieser Metalldünnschicht durch Einlagerung von Wasserstoff basiert. Diese Methoden weisen ebenfalls eine Minderung der Empfindlichkeit bei Wasserkondensatbildung sowie durch Kohlenmonoxidadsorption auf. Bei wasserstoffhaltigen Gasen wie NH₃ und H₂S tritt eine Verminderung der Detektionsfähigkeit durch Verringerung der Selektivität des Sensors auf.

Eine weitere etablierte Methode zur Messung von Wasserstoff in einem breiten Konzentrationsbereich stützt sich auf die deutlich geringere Leitfähigkeit von Wasserstoff im Vergleich zu anderen Gasen. Ein Nachteil dieser Methode ist jedoch die geringe Selektivität, wodurch es in Fällen, in denen wasserstoffhaltige Gase detektiert werden die weitere Gaskomponenten aufweisen zu Fehlergebnissen kommt.

US-5483228 offenbart eine Sicherheitsschaltung für Akkumulatoren, die mit katalytische Körper vorgesehen ist, die in dem zu überwachenden Gas angeordnet sind und Wasserstoff mit Sauerstoff zu Wasser rekombinieren; die dabei entstehende Wärme wird auf einen Bimetallschalter übertragen, der dann einen Detektionskreis unterbricht, wenn ein vorgegebenes Temperaturniveau erreicht wird - dadurch kann entweder ein Signal abgegeben oder der Ladevorgang unterbrochen werden.

DE-3151084 offenbart ein Verfahren zum Erkennen und Entfernen von Wasserstoff aus Flüssigkeitskreisläufen - der Wasserstoff wird über eine erste poröse Wand einem Wasserstoff-Sauerstoff-Kombinationskatalysator zugeführt, der über eine zweite poröse Wand mit der Umgebung in Verbindung steht; die bei der Rekombination entstehende Temperaturerhöhung dient der Erkennung des Vorhandenseins von Wasserstoff.

EP-1780826 offenbart einen Gas-Rekombinator für Akkumulatoren - der Gas-Rekombinator hat einen zylindrischen Katalysatorstab, ein poröses Keramikrohr, das den Katalysatorstab umgibt, und einen Absorber, der den Raum zwischen dem Keramikrohr und dem Katalysatorstab ausfüllt. Wasserstof und Sauerstoff, die bei der Ladung des Akkumulators entstehen, werden mittels des Rekombinators rekombiniert und als Wasser in die Akkumulatorzelle zurückgeführt.

Es ist daher die **Aufgabe** der vorliegenden Erfindung, eine Vorrichtung zur Detektion von Wasserstoff in einem Gasvolumen sowie ein entsprechendes Verfahren zur Detektion von Wasserstoff anzugeben.

**Gelöst** wird diese Aufgabe hinsichtlich der Vorrichtung durch eine Vorrichtung gemäss Anspruch 1 zur Detektion von Wasserstoff in einem Gasvolumen, aufweisend eine Einrichtung zur exothermen katalytischen Rekombination von Wasserstoff in Anwesenheit von Sauerstoff zu Wasser, eine Einrichtung zur Bestimmung der bei der Rekombination auftretenden Temperaturänderung, eine Einrichtung zum Vergleich der bei der Rekombination aufgetretenen Temperaturänderung mit einem hinterlegten Grenzwert, sowie eine Einrichtung zur Ausgabe eines Signals, wobei die Einrichtung zur Rekombination des Wasserstoffes mit der Einrichtung zur Bestimmung der bei der Rekombination auftretenden Temperaturänderung derart verbunden ist, dass eine im Wesentlichen fehlerfreie Bestimmung der bei der Rekombination auftretenden Temperaturänderung des Katalysators erfolgen kann und die Einrichtung zum Vergleich der bei der Rekombination auftretenden Temperaturänderung mit dem hinterlegten Grenzwert mit der Einrichtung zur Ausgabe eines Signals so verbunden ist, dass bei Überschreitung des hinterlegten Grenzwertes ein Signal ausgegeben wird. Vorteilhafter Weise ermöglicht es die erfindungsgemäße Vorrichtung sowohl Wasserstoff (Knallgas) zu detektieren, als auch den Wasserstoffanteil abzubauen, und zwar auf einen Wert unterhalb der Explosionsgrenze von 4 Vol.-%.

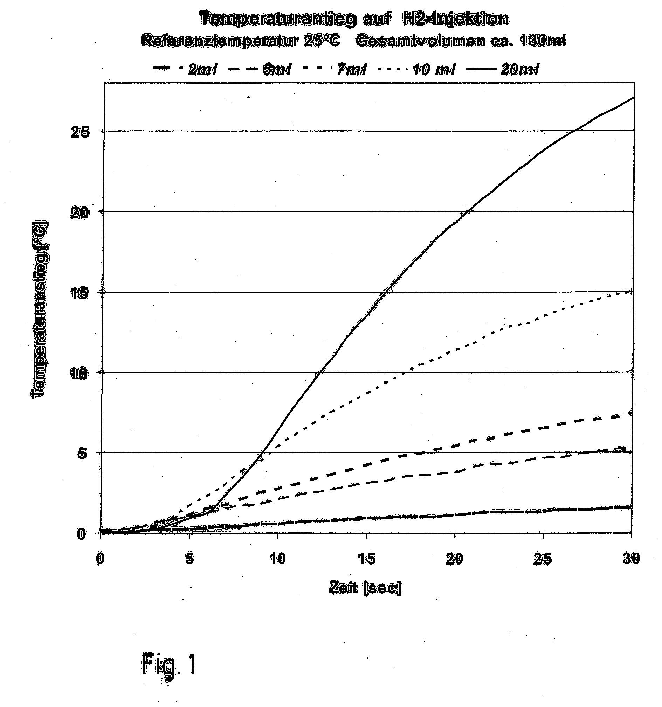

Das Funktionsprinzip der erfindungsgemäßen Einrichtung basiert hierbei auf der exothermen katalytischen Rekombination von Wasserstoff in Anwesenheit von Sauerstoff zu Wasser. Durch einen auftretenden Temperaturanstieg am Katalysator ermöglicht die Einrichtung den Nachweis von Wasserstoff. Die Einrichtung ermöglicht eine direkte Korrelation zwischen auftretender Wasserstoffkonzentration und Temperaturzunahme gegenüber einer Referenztemperatur (s. Fig. 1).

Durch die katalytische Rekombination des im Gasvolumen befindlichen Wasserstoffs mit Sauerstoff zu Wasser wird gleichzeitig zur Messung die Wasserstoffkonzentration gesenkt. Bei einer entsprechenden Ausgestaltung der erfindungsgemäßen Vorrichtung kann so die Wasserstoffkonzentration in einem Gasvolumen unter die Explosionsgrenze gesenkt und die von Wasserstoff ausgehende Gefahr vermieden werden.

Hinsichtlich des Verfahrens wird die Aufgabe der Erfindung durch ein Verfahren gemäss Anspruch 10 zur Detektion von Wasserstoff gelöst, aufweisend die Verfahrensschritte: Kontaktieren eines auf Wasserstoff zu detektierenden Gasvolumens mit einem Katalysator zur exothermen katalytischen Rekombination von Wasserstoff in Anwesenheit von Sauerstoff zu Wasser; Bestimmung des bei der exothermen katalytischen Rekombination auftretenden Temperaturanstieg am Katalysator; Vergleich des bestimmten Temperaturunterschiedes mit einem hinterlegten Grenzwert mittels einer geeigneten Einrichtung, wobei die Einrichtung bei Überschreiten des hinterlegten Grenzwertes ein Signal ausgibt.

Der in der Einrichtung zur katalytischen Rekombination von Wasserstoff eingesetzte Katalysator weist vorteilhafterweise ein Platinmetall, insbesondere Platin oder Palladium auf. Der Katalysator kann auf ein Trägermaterial aufgebracht sein.

In einer bevorzugten Ausgestaltung der Einrichtung zur katalytischen Rekombination von Wasserstoff ist der Katalysator zur Vermeidung von Kondensation getempert bzw. auf ein getempertes Trägermaterial aufgebracht.

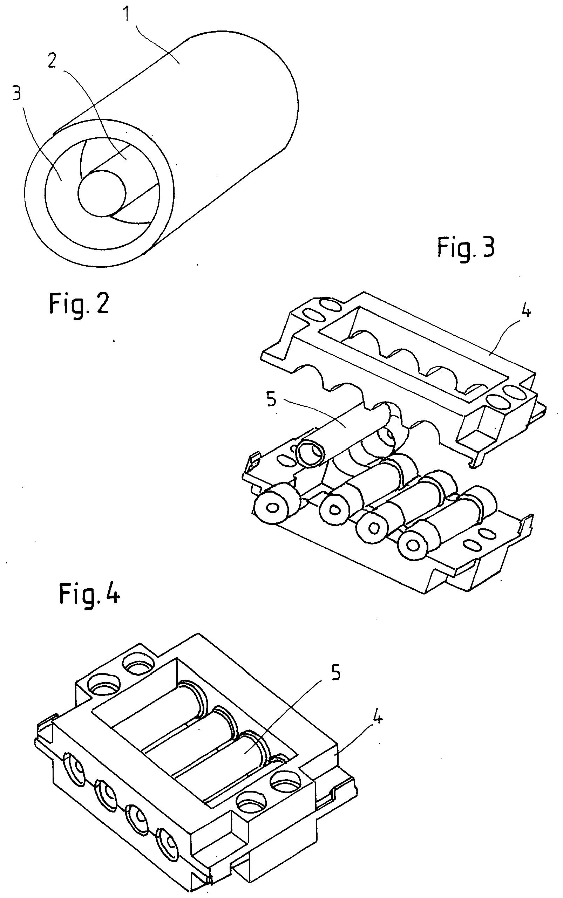

Vorteilhafterweise ist der ein Platinmetall aufweisende Katalysator mit einem porösen Material ummantelt (s. Fig.2). Das poröse Material ist vorteilhafterweise ein Keramikmaterial.

Das poröse Material selbst ist wiederum in einer vorteilhaften Ausgestaltung der Vorrichtung zur Detektion mit einem Absorptionsmaterial ummantelt. Hierbei dient das Absorptionsmaterial zur Absorption von Inhibitoren, welche als Katalysatorgift die reaktive Oberfläche des Katalysators beeinträchtigen können. Dementsprechend geeignete Absorptionsmaterialien sind beispielsweise Silberoxid, Eisenoxid, Kupferoxid oder Manganoxid sowie Mischungen dieser Oxide.

Zur Bestimmung des bei der Rekombination auftretenden Temperaturunterschiedes eignen sich Einrichtungen wie Thermometer, Temperatursensoren oder Thermoelemente.

Die Einrichtung zum Vergleich des bei der Rekombination auftretenden Temperaturunterschiedes mit einem hinterlegten Grenzwert kann vorteilhafterweise eine Vergleichschaltung oder eine Rechnereinheit sein.

Die Ausgestaltung in der Form, dass das Katalysatormaterial der Einrichtung zur katalytischen Rekombination mit einem porösen Material wie einem Keramikmaterial ummantelt ist, welches wiederum mit einem Absorptionsmaterial der zuvor genannten Art ummantelt ist stellt sicher, dass im Falle von Feuchtigkeitsabsorption durch das hydrophobierte Absorptionsmaterial der Katalysator in seiner katalytischen Wirkung nicht beeinträchtigt wird. Darüber hinaus werden, wie zuvor beschrieben, die an die katalytische Oberfläche der Einrichtung zur Rekombination von Wasserstoff vordringenden Gase in der Art eines Filters gereinigt und so von Störstoffen wie beispielsweise Hydriden oder Schwefelverbindungen befreit. Hydride oder Schwefelverbindungen können als Katalysatorgift die Aktivität der als Katalysator eingesetzten Platinmetalle herabsetzen und somit die Wirkungsweise der Detektionsvorrichtung beeinträchtigen.

Das gegebenenfalls wasserstoffhaltige zu detektierende Gas, was darüber hinaus Sauerstoff, beispielsweise in Form von Luftsauerstoff, aufweist kann mittels geeigneter Einrichtungen zur Förderung und zum Transport von Gasen wie Pumpen oder Ventilatoren an die Katalysatoroberfläche der Einrichtung zur katalytischen Rekombination durch das Absorptionsmaterial und das poröse Material hindurch herangeführt werden. Alternativ kann das Gas lediglich durch Diffusion an die katalytische Oberfläche herantreten.

An der katalytischen Oberfläche selbst wird der eventuell im zu detektierenden Gasvolumen befindliche Wasserstoff in Anwesenheit von Sauerstoff katalytisch zu Wasser rekombiniert. Bei dieser Rekombination handelt es sich um eine stark exotherme Reaktion, bei welcher ca. 280 kJ/mol Wasserstoff an Energie frei wird.

Diese Energie wird in Form von Wärmeenergie freigesetzt. Dies führt zu einer Temperaturveränderung an der katalytischen Oberfläche der Einrichtung zur Rekombination. Das bei der Rekombination entstehende Wasser kann in Form von Wasserdampf abgeführt werden.

Es hat sich herausgestellt, dass auf Grund der bei der katalytischen Rekombination von Wasserstoff freigesetzten großen Energiemenge sich diese Energiefreisetzung als empfindliche Sonde zur Detektion von Wasserstoff in Gasvolumina eignet.

Gemäß der vorliegenden Erfindung wird die durch die katalytische Rekombination freigesetzte Energiemenge durch Messung einer relativen Temperaturänderung ΔT an der Katalysatoroberfläche bestimmt. Durch die Messung eines relativen Temperaturunterschiedes sind die erfindungsgemäße Vorrichtung sowie das erfindungsgemäße Verfahren in vorteilhafter Weise nahezu unbeeinflusst von der Umgebungstemperatur.

Die bei der katalytischen Rekombination ermittelte relative Temperaturänderung kann in einer Vergleichsschaltung oder einer Rechnereinheit mit einem hinterlegten Grenzwert verglichen werden. Hierbei entspricht der Grenzwert der Temperaturänderung einer Temperaturänderung, wie sie durch eine als Grenze zu definierende Wasserstoffkonzentration im Gasvolumen an der Einrichtung zur katalytischen Rekombination hervorgerufen wird.

Die heute zur Verfügung stehenden Einrichtungen zur Bestimmung von Temperaturen umfassen Temperatursensoren wie digitale (elektronische) Thermometer, Widerstandsthermometer, IR-Sensoren oder Thermoelemente und weisen eine sehr große Genauigkeit auf. Darüber hinaus sind sie unempfindlich gegenüber Korrosion oder andere aggressive Einflüsse.

Stellt die Einrichtung zum Vergleich des bei der Rekombination auftretenden Temperaturunterschiedes mit dem hinterlegten Grenzwert eine Überschreitung des Temperaturunterschiedgrenzwertes fest, kann diese ein entsprechendes Signal mittels einer Ausgabeeinrichtung ausgeben. Dabei kann das Signal ein Steuersignal zur Ansteuerung eines optischen und/oder akustischen Signalgebers sein, welcher in Form einer Warnleuchte oder einer Warnsirene geeignet ist, Personen über eine entsprechende Wasserstoffkonzentration, wie sie dem hinterlegten Grenzwert entspricht, zu informieren.

Darüber hinaus kann mit dem durch die Ausgabeeinrichtung ausgegebenen Steuersignal ein entsprechendes Folgeereignis der Grenzwertüberschreitung an Wasserstoff ausgelöst werden, wie beispielsweise das Schließen von Ventilen in wasserstoffführenden Leitungen, um die Zufuhr von Wasserstoff zu Einrichtungen zu unterbinden oder die Inbetriebnahme von entsprechenden Lüftungseinrichtungen zur Belüftung von Einrichtungen oder Anlagen, um die darin befindlichen Wasserstoffkonzentrationen unter einen entsprechenden Grenzwert abzusenken. Selbstverständlich liegt es im Rahmen der Erfindung, mit Hilfe des über die Ausgabeeinrichtung ausgegebenen Steuersignals andere Einrichtungen in Abhängigkeit des detektierten Wasserstoffs zu steuern.

In der Ausgestaltung der erfindungsgemäßen Vorrichtung weist diese eine Heizeinrichtung zum Temperieren der Einrichtung zur katalytischen Rekombination des Wasserstoffes auf. Hierdurch kann die Ablagerung von Kondensfeuchtigkeit in der Einrichtung vermieden werden und die Betriebsbereitschaft der Vorrichtung zur Detektion von Wasserstoff über weite Betriebsbedingungen sichergestellt werden.

In einer weiteren vorteilhaften Ausgestaltung sind die Einrichtung zur katalytischen Rekombination und die Einrichtung zum Vergleich der bei der Rekombination auftretenden Temperaturänderung mit einem hinterlegten Grenzwert räumlich so voneinander abgegrenzt, dass ein Einbringen von Wasserstoffgas in die Einrichtung zum Vergleich, welche eine Vergleichsschaltung oder eine Rechnereinheit sein kann, vermieden wird, um etwaige Zündreaktionen des Wasserstoffs durch elektrische Funken zu vermeiden.

Die erfindungsgemäße Vorrichtung zur Detektion von Wasserstoff lässt sich auf Basis der heute zur Verfügung stehenden Miniaturisierung von elektronischen Schaltungen als komplexe Einrichtung fertigen, welche nur einen geringen Raumbedarf hat. Hierdurch lassen sich die erfindungsgemäßen Vorrichtungen in vorteilhafter Weise in entsprechende wasserstoffbetriebene oder wasserstoffführende Systeme wie Brennstoffzellen, Wasserstoffkompressoren, Wasserstofftankstellen, wasserstoffbetriebene Fahrzeuge und dergleichen integrieren. Die Kopplung der erfindungsgemäßen Vorrichtung mit den Steuereinrichtungen der zuvor beschriebenen Systeme führt somit zu einer deutlichen Verbesserung der Betriebssicherheit der Systeme.

Darüber hinaus lässt sich die erfindungsgemäße Vorrichtung auch zur manuellen Detektion von Leckagestellen an Leitungssystemen oder Einrichtungen nutzen, indem die Vorrichtung in ein entsprechendes Detektionsgerät integriert wird. Hierbei kann das Detektionsgerät eine Pumpe zum Ansaugen von Gasvolumina über eine entsprechende Sonde aufweisen, wobei die Pumpe das angesogene Gasvolumen in eine erfindungsgemäße Vorrichtung zur Detektion leitet. Bei Überschreiten eines entsprechenden Grenzwertes einer Wasserstoffkonzentration kann dann ein geeignetes Signal ausgegeben werden. Hierbei kann die Grenzwertkonzentration durch Variation des Grenzwertes für die Temperaturveränderung entsprechend variiert werden. Letztendlich ist auch eine Korrelation zwischen der Temperatur an der Katalysatoroberfläche und einer aktuellen Wasserstoffkonzentration möglich, so dass die erfindungsgemäße Vorrichtung auch zur Integration in ein System zur Messung der Wasserstoffkonzentration in einem Gasvolumen dienen kann.

Auf Grund der Möglichkeit, entsprechend erfindungsgemäße Vorrichtungen in komplexer Bauweise auszuführen, ist es erfindungsgemäß auch vorgesehen, mehrere erfindungsgemäße Vorrichtungen zu vernetzen, um auch größere wasserstoffführende Systeme oder Einrichtungen hinsichtlich etwaiger Leckagen überwachen zu können (Fig. 3 und 4).
Fig. 1 zeigt ein Diagramm zum Temperaturanstieg des Katalysators als Funktion der Zeit in Abhängigkeit der H₂ Konzentration.
Fig. 2 zeigt den Aufbau einer Detektorvorrichtung
Fig. 3 zeigt den Aufbau einer vernetzten Vorrichtungseinheit.
Fig. 4 zeigt eine vernetzte Vorrichtungseinheit.

Fig. 1 zeigt den Temperaturverlauf an einer Katalysatoroberfläche zur katalytischen Reformation von Wasserstoff und Sauerstoff zu Wasser. In Abhängigkeit der Wasserstoffkonzentration im zu untersuchenden Gasvolumen wird ein Temperaturanstieg an der Oberfläche des Katalysators beobachtet. Im vorliegenden Fall wurde der Temperaturverlauf einer Platinmetalloberfläche bei der Exposition einer Wasserstoffkonzentration von jeweils ca. 1,5 Vol.-%, 3,9 Vol.-%, 5,4 Vol.-%, 7,8 Vol.-% und 15,4 Vol.-% beobachtet. Die zugrunde liegende Basistemperatur lag bei ca. 25°C. Es wurde ein Gasvolumen von ca.130ml mit jeweils 2ml, 5ml, 7ml, 10ml oder 20ml H₂ beaufschlagt. Hierbei wurde nach 30 Sekunden eine Temperaturerhöhung von ca. 1,5°C, 5°C, 7,5°C, 15°C bzw. 27°C beobachtet. Ist erfindungsgemäß ein Grenzwert von 1,5°C hinterlegt und wird dieser Grenzwert an der Katalysatoroberfläche innerhalb eines Messzyklus von z.B. 30 Sekunden überschritten, liegt eine H₂-Konzentration >2 Vol.-% vor. Es ist somit als eine sichere und weitgehend äußeren Einflüssen gegenüber unempfindliche Bestimmung der Wasserstoffkonzentration in Gasvolumina möglich: In vorteilhafter Weise führt die erfindungsgemäße Vorrichtung zu einer Reduzierung der Wasserstoffkonzentration im Gasvolumen durch die exotherme Rekombinationsreaktion bis unterhalb der Explosionsgrenze.

Fig. 2 zeigt den Aufbau einer Detektorvorrichtung. Hierbei ist 1 eine poröse Keramikummantelung, 2 ein Katalysatorstift und 3 der Absorberraum. Der Katalysatorstift 1 ist von einem porösen Keramikmaterial 2 ummantelt, wobei dieses die Katalysatoroberfläche vor störenden Einflüssen schützen soll. Zwischen Katalysatorstift 1 und dem Absorbermaterial bildet sich ein Absorberraum 3 aus, in welchen das zu detektierende Gasgemisch durch das Absorbermaterial 2 eindringen kann und dort an der Oberfläche des Katalysatorstiftes 1 reagieren kann.

Fign. 3 und 4 zeigen eine erfindungsgemäß vernetzte Vorrichtung zur Detektion von Wasserstoff, bei welcher mehrere Detektionselemente 5, welche jeweils aus einem Katalysatorstift 1, einem Keramikmaterial 2 und einem Absorberraum 3 bestehen, in einem gemeinsamen Aufnahme 4 aufgenommen werden. Hierdurch erhöht sich das Rekombinationsvolumen der vernetzten Einheit und es erfolgt ein schnellerer Abbau der Wasserstoffkonzentration durch katalytische Rekombination. Dies trägt wiederum zur Erhöhung der Betriebssicherheit von Wasserstoffanlagen bei.

### Bezugszeichenliste:

- 1: Katalysatorstift
- 2: Keramikmaterial
- 3: Absorberraum
- 4: Aufnahme
- 5: Detektionselement

## Patentansprüche

1. Vorrichtung zur Detektion von Wasserstoff in einem Gasvolumen und zur Reduzierung der Wasserstoffkonzentration in einem Luft/Wasserstoff-Gasgemisch bis unterhalb der Explosionsgrenze, aufweisend
- eine Einrichtung zur exothermen katalytischen Rekombination von Wasserstoff in Anwesenheit von Sauerstoff in einem Luft/Wasserstoff-Gasgemisch zu Wasser;
- eine Einrichtung zur Bestimmung der bei der Rekombination auftretenden Temperaturänderung;
- eine Einrichtung zum Vergleich der bei der Rekombination aufgetretenen Temperaturänderung mit einem hinterlegten Grenzwert;
- eine Ausgabeeinrichtung zur Ausgabe eines Signals,
- wobei die Einrichtung zur Rekombination des Wasserstoffes mit der Einrichtung zur Bestimmung der bei der Rekombination auftretenden Temperaturänderung derart verbunden ist, dass eine Bestimmung der bei der Rekombination auftretenden Temperaturänderung an der Katalysatoroberfläche der Einrichtung zur exothermen katalytischen Rekombination erfolgen kann und die Einrichtung zum Vergleich der bei der Rekombination auftretenden Temperaturänderung mit dem hinterlegten Grenzwert mit der Ausgabeeinrichtung so verbunden ist, dass bei Überschreitung eines hinterlegten Grenzwertes ein Signal ausgegeben wird, wobei mehrere Einrichtungen zur exothermen katalytischen Rekombination von Wasserstoff in Anwesenheit von Sauerstoff zu Wasser als Detektionselemente (5) vorgesehen sind und, wobei die Ausgabeeinrichtung ein Steuersignal ausgibt, mit welchem ein Ventil oder eine Lüftungseinrichtung ansteuerbar ist,
- eine gemeinsame Aufnahme (4), in der die Detektionselemente (5) aufgenommen sind, wobei die Detektionselemente (5) jeweils einen Katalysatorstift (2), eine poröse Keramikummantelung (1) und einen Absorberraum (3) umfassen, wobei der Katalysatorstift (2) von der porösen Keramikummantelung (1) ummantelt und zwischen dem Katalysatorstift (2) und der porösen Keramikummantelung (1) der Absorberraum (3) ausgebildet ist; sowie
- eine Heizeinrichtung zum Temperieren der Detektionselemente (5).

2. Vorrichtung nach Anspruch 1, **dadurch gekennzeichnet, dass** die Einrichtung zur katalytischen Rekombination als Katalysator ein Platinmetall, insbesondere Platin oder Palladium aufweist.

3. Vorrichtung nach einem der Ansprüche 1 oder 2, **dadurch gekennzeichnet, dass** die Ummantelung aus porösem Material mit einer Ummantelung aus einem Absorptionsmaterial ummantelt ist.

4. Vorrichtung nach Anspruch 3, **dadurch gekennzeichnet, dass** das Absorptionsmaterial Silberoxid, Eisenoxid, Kupferoxid oder Magnesiumoxid oder eine Mischung dieser aufweist.

5. Vorrichtung nach einem der Ansprüche 2 bis 4, **dadurch gekennzeichnet, dass** der Katalysator in der Einrichtung zur katalytischen Rekombination auf einem Trägermaterial aufgebracht ist.

6. Vorrichtung nach Anspruch 5, **dadurch gekennzeichnet, dass** das Trägermaterial ein Silizium Aluminiumoxid, γ-Al₂O₃ oder SiO₂ (amorphes Silikat) Material ist.

7. Vorrichtung nach einem der vorhergehenden Ansprüche, **dadurch gekennzeichnet, dass** die Einrichtung zur Bestimmung des bei der Rekombination auftretenden Temperaturunterschiedes ein Thermometer, ein Temperatursensor oder ein Thermoelement ist.

8. Vorrichtung nach einem der vorhergehenden Ansprüche, **dadurch gekennzeichnet, dass** die Einrichtung zum Vergleich des bei der Rekombination auftretenden Temperaturunterschiedes mit einem hinterlegten Grenzwert eine Vergleichsschaltung oder eine Rechnereinheit ist.

9. Vorrichtung nach einem der vorhergehenden Ansprüche, **dadurch gekennzeichnet, dass** die Ausgabeeinrichtung, ein Steuersignal ausgibt, mit welchem ein optischer und/oder akustischer Signalgeber ansteuerbar ist.

10. Verfahren zur Detektion von Wasserstoff und zur Reduzierung der Wasserstoffkonzentration im Gasvolumen bis unterhalb der Explosionsgrenze, mittels einer Vorrichtung gemäß einem der Ansprüche 1 bis 9 aufweisend die Verfahrensschritte:
- Kontaktieren eines auf Wasserstoff zu detektierenden Luft/Wasserstoff-Gasgemisches mit dem Katalysator mehrerer in der gemeinsamen Aufnahme (4) aufgenommener Detektionselemente (5) in Anwesenheit von Sauerstoff zur exothermen katalytischen Rekombination von Wasserstoff und Sauerstoff zu Wasser;
- Bestimmung des bei der exothermen katalytischen Rekombination auftretenden Temperaturunterschiedes am Katalysator;
- Vergleichen des bestimmten Temperaturunterschiedes mit einem hinterlegten Grenzwert mittels einer geeigneten Einrichtung, wobei die Ausgabeeinrichtung bei Überschreiten des hinterlegten Grenzwertes ein Steuersignal an ein Ventil oder eine Lüftungseinrichtung ausgibt, wobei mittels des Ventils die Wasserstoffzufuhr zu den Einrichtungen unterbunden oder mittels der Lüftungseinrichtung die Einrichtungen belüftet werden, und wobei die Detektionselemente (5) mittels der Heizeinrichtung temperiert werden.

11. Verfahren nach Anspruch 13, **dadurch gekennzeichnet, dass** als Katalysator ein Platinmetall, vorzugsweise Palladium oder Platin verwendet wird.

12. Verfahren nach einem der Ansprüche 10 oder 11, **dadurch gekennzeichnet, dass** zur Bestimmung des bei der exothermen katalytischen Rekombination am Katalysator auftretenden Temperaturunterschiedes ein Thermometer, ein Temperatursensor oder ein Thermoelement verwendet wird.

13. Verfahren nach einem der Ansprüche 10 bis 12, **dadurch gekennzeichnet, dass** zum Vergleich des bestimmten Temperaturunterschiedes mit einem hinterlegten Grenzwert eine Vergleichsschaltung oder eine Rechnereinheit genutzt wird.

## Claims

1. A device for detecting hydrogen in a gas volume and reducing the hydrogen concentration in an air-hydrogen-gas mixture to beneath the explosion limit, comprising:
- a device for the exothermal catalytic recombination of hydrogen in the presence of oxygen in an air-hydrogen-gas mixture for obtaining water;
- a device for determining the temperature change that occurs during the recombination;
- a device for comparing the temperature change that has taken place during the recombination with a stored limit value;
- an output device for emitting a signal,
- wherein the device for the recombination of the hydrogen is connected to the device for determining the temperature change that occurs during the recombination in such a way that the temperature change that occurs during the recombination can be determined on the catalyst surface of the device for the exothermal catalytic recombination and the device for comparing the temperature change that has taken place during the recombination with the stored limit value is connected to the output device such that if a stored limit value is exceeded, a signal will be output, wherein several devices for the exothermal catalytic recombination of hydrogen in the presence of oxygen for obtaining water are provided as detection elements (5), and wherein the output device emits a control signal, by means of which a valve or a ventilation device can be controlled, a common receiving space (4) being provided, in which the detection elements (5) are received, wherein the detection elements (5) each comprise a catalytic pin (2), a porous ceramic jacket (1) and an absorber space (3), wherein the catalytic pin (2) is sheathed by the porous ceramic jacket (1) and the absorber space (3) is formed between the catalytic pin (2) and the porous ceramic jacket (1); as well as a heating device is provided for tempering the detection elements (5).

2. A device according to claim 1, **characterized in that** the device for the catalytic recombination, as a catalyst, comprises a platinum metal, in particular platinum or palladium.

3. A device according to one of the claims 1 or 2, **characterized in that** the jacket made of porous material is sheathed by a coating made of an absorption material.

4. A device according to claim 3, **characterized in that** the absorption material comprises silver oxide, iron oxide, copper oxide or magnesium oxide or a mixture of these ones.

5. A device according to one of the claims 2 through 4, **characterized in that** the catalyst in the device for the catalytic recombination is applied on a carrier material.

6. A device according to claim 5, **characterized in that** the carrier material is a silicon aluminium oxide, γ-Al₂O₃ or SiO₂ (amorphous silicate) material.

7. A device according to one of the preceding claims, **characterized in that** the device for determining the temperature change that occurs during the recombination is a thermometer, a temperature sensor or a thermal element.

8. A device according to one of the preceding claims, **characterized in that** the device for comparing the temperature change that has taken place during the recombination with a stored limit value is a comparator circuit or a computer unit.

9. A device according to one of the preceding claims, **characterized in that** the output device emits a control signal, by means of which an optical and/or acoustic signal generator can be controlled.

10. A method for detecting hydrogen and reducing the hydrogen concentration in in the gas volume to beneath the explosion limit by means of a device according to one of the claims 1 through 9, comprising the process steps:
- bringing an air-hydrogen-gas mixture to be detected with respect to hydrogen into contact with the catalyst of several detection elements (5) received in the common receiving space (4) in the presence of oxygen for the exothermal catalytic recombination of hydrogen and oxygen to obtain water;
- determining the temperature change that occurs during the exothermal catalytic recombination at the catalyst;
- comparing the determined temperature change with a stored limit value by means of an appropriate device, wherein the output device will send a control signal to a valve or a ventilation device, if the stored limit value is exceeded, wherein the hydrogen supply to the devices will be stopped by means of the valve or the devices will be ventilated by means of the ventilation device, and wherein the detection elements (5) will be tempered by means of the heating device.

11. A method according to claim 10, **characterized in that** a platinum metal, in particular platinum or palladium will be used as catalyst.

12. A method according to one of the claims 10 or 11, **characterized in that** a thermometer, a temperature sensor or a thermal element will be used for determining the temperature change that occurs during the exothermal catalytic recombination at the catalyst.

13. A method according to one of the claims 10 through 12, **characterized in that** a comparator circuit or a computer unit will be used for comparing the determined temperature change with a stored limit value.

## Revendications

1. Dispositif de détection d'hydrogène dans un volume de gaz et de réduction de la concentration d'hydrogène dans un mélange gazeux se composant d'air et d'hydrogène jusqu'en dessous de la limite explosive, comprenant :
- un dispositif destiné à la recombinaison catalytique exothermique d'hydrogène en présence d'oxygène dans un mélange gazeux se composant d'air et d'hydrogène pour obtenir de l'eau ;
- un dispositif destiné à déterminer le changement de température qui se produit pendant la recombinaison ;
- un dispositif destiné à comparer le changement de température, qui s'est produit pendant la recombinaison, avec une valeur limite mémorisée ;
- un dispositif de sortie pour émettre un signal,
- dans lequel le dispositif destiné à la recombinaison de l'hydrogène est relié au dispositif destiné à déterminer le changement de température qui se produit pendant la recombinaison, de sorte que le changement de température qui se produit pendant la recombinaison peut être déterminé sur la surface du catalyseur du dispositif destiné à la recombinaison catalytique exothermique et le dispositif destiné à comparer le changement de température, qui s'est produit pendant la recombinaison, avec la valeur limite mémorisée est relié au dispositif de sortie, de sorte que si une valeur limite est excédée, un signal sera émis, plusieurs dispositifs destinés à la recombinaison catalytique exothermique d'hydrogène en présence d'oxygène pour obtenir de l'eau étant prévus comme des éléments de détection (5), le dispositif de sortie émettant un signal de commande, par moyen duquel une soupape ou un dispositif de ventilation peut être commandé, dans lequel un logement commun (4) est prévu, dans lequel les éléments de détection (5) sont reçus, les éléments de détection (5) comprenant chacun une tige de catalyseur (2), une gaine en céramique poreuse (1) et un espace d'absorption (3), la tige de catalyseur (2) étant enveloppée par la gaine en céramique poreuse (1) et l'espace d'absorption (3) étant formé entre la tige de catalyseur (2) et la gaine en céramique poreuse (1) ; ainsi qu'un dispositif de chauffage étant prévu pour tempérer les éléments de détection (5).

2. Dispositif selon la revendication 1, **caractérisé en ce que** le dispositif destiné à la recombinaison catalytique comprend en tant que catalyseur un métal du groupe du platine, notamment du platine ou du palladium.

3. Dispositif selon l'une des revendications 1 ou 2, **caractérisé en ce que** la gaine en matériau poreux est enrobée par une enveloppe en un matériau d'absorption.

4. Dispositif selon la revendication 3, **caractérisé en ce que** le matériau d'absorption comprend de l'oxyde d'argent, de l'oxyde de fer, de l'oxyde de cuivre ou de l'oxyde de magnésium ou un mélange de ceux-ci.

5. Dispositif selon l'une des revendications 2 à 4, **caractérisé en ce que** le catalyseur dans le dispositif destiné à la recombinaison catalytique est appliqué sur un matériau de support.

6. Dispositif selon la revendication 5, **caractérisé en ce que** le matériau de support est un matériau en oxyde d'aluminium de silicium, en γ-Al₂O₃ ou en SiO₂ (silicate amorphe).

7. Dispositif selon l'une des revendications précédentes, **caractérisé en ce que** le dispositif destiné à déterminer le changement de température qui se produit pendant la recombinaison est un thermomètre, un capteur de température ou un élément thermique.

8. Dispositif selon l'une des revendications précédentes, **caractérisé en ce que** le dispositif destiné à comparer le changement de température, qui s'est produit pendant la recombinaison, avec une valeur limite mémorisée est un circuit de comparaison ou une unité de traitement.

9. Dispositif selon l'une des revendications précédentes, **caractérisé en ce que** le dispositif de sortie émet un signal de commande, par moyen duquel un émetteur de signaux optique et/ou acoustique peut être commandé.

10. Procédé de détection d'hydrogène et de réduction de la concentration d'hydrogène dans le volume de gaz jusqu'en dessous de la limite explosive par moyen d'un dispositif selon l'une des revendications 1 à 9, comprenant les étapes de procédé suivantes de :
- mettre en contact un mélange gazeux se composant d'air et d'hydrogène et étant à détecter par rapport à l'hydrogène avec le catalyseur des plusieurs éléments de détection (5) reçus dans le logement commun (4) en présence d'oxygène pour la recombinaison catalytique exothermique d'hydrogène et d'oxygène pour obtenir de l'eau ;
- déterminer le changement de température qui se produit pendant la recombinaison catalytique exothermique sur le catalyseur ;
- comparer le changement de température déterminé avec une valeur limite mémorisée par moyen d'un dispositif approprié, le dispositif de sortie émettant un signal de commande à une soupape ou à un dispositif de ventilation, si la valeur limite mémorisée est excédée, l'alimentation en hydrogène aux dispositifs étant coupée par moyen de la soupape ou les dispositifs étant ventilés par moyen du dispositif de ventilation, et les éléments de détection (5) étant tempérés par moyen du dispositif de chauffage.

11. Procédé selon la revendication 13, **caractérisé en ce qu'**on utilise un métal du groupe du platine, notamment du palladium ou du platine en tant que catalyseur.

12. Procédé selon l'une des revendications 10 ou 11, **caractérisé en ce qu'**on utilise un thermomètre, un capteur de température ou un élément thermique pour déterminer le changement de température qui se produit pendant la recombinaison catalytique exothermique sur le catalyseur.

13. Procédé selon l'une des revendications 10 à 12, **caractérisé en ce qu'**on utilise un circuit de comparaison ou une unité de traitement pour comparer le changement de température déterminé avec une valeur limite mémorisée.
